# EUROPÄISCHE PATENTANMELDUNG

(11) **EP 1 886 650 A1**
(43) Veröffentlichungstag der Anmeldung: **13.02.2008**
(21) Anmeldenummer: 07013427.5
(22) Anmeldetag: 10.07.2007
(51) Int. Cl.: A61F 2/90, B23K 26/38, B26F 3/00

(54) **Endoprothese und Verfahren zur Herstellung einer solchen**

(30) Priorität: 07.08.2006 DE 102006038232
(71) Anmelder: BIOTRONIK VI Patent AG, 6341 Baar (CH)
(72) Erfinder: Barthel, Harald, 91054 Erlangen (DE); Fringes, Matthias, 91522 Ansbach (DE); Gerold, Bodo, Dr., 91225 Zellingen (DE); Riedmüller, Johannes, 90411 Nürnberg (DE)
(74) Vertreter: Lindner-Vogt, Karin L.

(57) **Zusammenfassung**

Die Erfindung betrifft eine Endoprothese, insbesondere intraluminale Endoprothese, z.B. ein Stent, mit einem Grundgitter (2) und einem an einer Trägerstruktur (6, 6', 6", 16, 16', 26, 26', 36, 46, 56, 66) befestigten Funktionselement (8, 8', 18, 18', 28, 28', 38, 48, 58, 68), das zumindest in einem Teil seines Volumens eine verglichen mit dem Material des Grundgitters (2) verschiedene Materialzusammensetzung aufweist. Die Trägerstruktur (6, 6', 6", 16, 16', 26, 26', 36, 46, 56, 66) ist an ihrem ersten, im Wesentlichen fingerförmigen Ende an dem Grundgitter (2) angeordnet und ragt von diesem im Wesentlichen fortsatzartig weg. Das Funktionselement (8, 8', 18, 18', 28, 28', 38, 48, 58, 68) ist an einem von dem Grundköper wegragenden Bereich der Trägerstruktur (6, 6', 6", 16, 16', 26, 26', 36, 46, 56, 66) angeordnet und umgibt diesen Bereich mindestens teilweise.

## Beschreibung

Die Erfindung betrifft eine Endoprothese, insbesondere eine intraluminale Endoprothese, z.B. einen Stent, mit einem Grundgitter und einem an einer Trägerstruktur befestigten Funktionselement, das zumindest in einem Teil seines Volumens eine verglichen mit dem Material des Grundgitters verschiedene Materialzusammensetzung aufweist. Die Erfindung betrifft außerdem ein Verfahren zur Herstellung einer derartigen Endoprothese.

Stents sind endovaskuläre Prothesen, die zur Behandlung von Stenosen (Gefäßverengungen) eingesetzt werden können. Sie weisen ein hohlzylinder- oder röhrenförmiges Grundgitter auf, das an beiden Längsenden der Röhren offen ist. Das röhrenförmige Grundgitter einer derartigen Endoprothese wird in das zu behandelnde Gefäß eingesetzt und dient dazu, das Gefäß zu stützen.

Derartige Stents nehmen üblicherweise zwei Zustände an, nämlich einen komprimierten Zustand mit einem kleinen Durchmesser und einen expandierten Zustand mit einem größeren Durchmesser. Im komprimierten Zustand kann der Stent mittels eines Katheters in das zu stützende Gefäß eingeführt und an der zu behandelnden Stelle positioniert werden. Am Ort der Behandlung wird der Stent dann beispielsweise mittels eines Ballonkatheters dilatiert bzw. geht (bei Verwendung eines Formgedächtnis-Metalls als Stentmaterial) durch die Erwärmung im Blut über seine Sprungtemperatur von selbst in den expandierten Zustand über. Aufgrund der Durchmesseränderung ist das Grundgitter des Stents einer starken mechanischen Belastung ausgesetzt.

Der als ein Gittergerüst ausgebildete Grundkörper des Stents, das üblicherweise aus einem metallischen Material, einer Keramik und/oder Kunststoff besteht, ist außerdem Zug- und Druckspannungen dadurch ausgesetzt, dass sich der Stent nach dem Einsetzen in das Gefäß an die Form und die Bewegung des Gefäßes anpasst.

Es ist bekannt, Stents mit Funktionselementen zu versehen, die zumindest in einem Teil ihres Volumens eine verglichen mit dem Material des Grundgitters verschiedene Materialzusammensetzung aufweisen. Diese Funktionselemente dienen beispielsweise der Bestimmung der Position eines Stents im Körper oder dem Freisetzen von Medikamenten.

Die Ermittlung der Position eines Stents geschieht häufig mittels bildgebender Verfahren, beispielsweise mittels einer Röntgenstrahleinrichtung. Da die für das Grundgitter von Stents verwendeten Materialien in der Regel Röntgenstrahlung nur in geringem Maße absorbieren, d.h. röntgen- oder radioluzent sind, werden Stents häufig mit sogenannten Röntgenmarkern versehen, die ein Material enthalten, das eine höhere Absorption der Röntgenstrahlen aufweist (röntgenopakes oder radioopakes Material).

Aus der Druckschrift DE 103 17 241 A1 ist ein Stent mit einem metallischen, radioluzenten Grundgitter und Markerelementen bekannt, die radioopakes Material aufweisen. Mittels Ausschneiden werden in Stegen des Grundgitters des bekannten Stents Ausnehmungen vorgesehen, die als Trägerstruktur wirken und in die später Markerelemente eingeschweißt und von einer Deckschicht aus Siliziumkarbid umschlossen werden. Eine weitere, in dieser Druckschrift offenbarte, allerdings kostenaufwändige Möglichkeit besteht darin, das Grundgitter vollständig aus einem Nitinoldraht mit einer Gold-Seele, die als Röntgenmarker dient, zu fertigen. Dieser mit der Gold-Seele versehene Nitinoldraht bildet den gesamten Endabschnitt des Stents und ist mit dem Grundgitter durch Schweißen verbunden.

Eine ähnliche Lösung wird auch in der Druckschrift DE 100 64 569 A1 beschrieben. Das in dieser Druckschrift offenbarte Verfahren zum Anbringen eines Markerelements an einen Stent sieht vor, dass ein zu verfestigendes, fließ- oder schüttfähiges Material oder Materialgemisch, beispielsweise in Form eines Granulats, in eine Ausnehmung am Grundgitter des Stents eingebracht und dort verfestigt wird. Die Verfestigung dieses Materials erfolgt beispielsweise mittels Sintern.

Aus der Druckschrift US 6,174,329 B1 ist ein beschichteter Stent bekannt, dessen Grundgitter radioluzent ist und der vollständig oder teilweise mit einer radioopaken Schicht versehen ist. Ferner kann eine Schutzschicht vorgesehen sein, die den beschichteten Stent vor Kratzern oder galvanischer Korrosion schützt und die Blut- und Biokompatibilität des Stents erhöht. Bei einer teilweisen Beschichtung werden die radioopaken Materialien an den geraden und nicht an den gekrümmt verlaufenden Abschnitten des Stents angebracht, da die gekrümmt verlaufenden Abschnitte bei der Expansion stärkeren mechanischen Belastungen ausgesetzt sind.

Schließlich wird in der Druckschrift US 6,293,966 B1 ein Stent mit radioopaken Markerelementen offenbart, der an seinen distalen bzw. proximalen Enden C-förmig ausgebildete Elemente aufweist, die jeweils eine im Wesentlichen kugelförmige Aufnahme ausbildet. Diese Aufnahmen dienen zum Einsetzen von Markerelementen mit kugelförmigen Endabschnitten. Die kugelförmigen Endabschnitte werden formschlüssig und ggf. mittels einer Schweißverbindung in den durch die C-förmigen Elemente gebildeten Aufnahmen befestigt.

Bei den bekannten Stents besteht nach wie vor das Problem, dass die direkt an dem Gittergerüst des Stents angebrachten Funktionselemente mit den röntgenopaken Materialien bei der Expansion und der Anpassung an die Gefäßgeometrie aufgrund des Materialunterschieds zwischen Grundgitter und Funktionselement durch die Kerbwirkung des Funktionselements Spannungsspitzen im Grenzbereich zwischen beiden Materialien auftreten und deshalb in diesem Bereich Defekte auftreten können.

Außerdem können bei der Stent-Positionsbestimmung mittels röntgenopaker Marker als Funktionselemente Fehler bei den Messungen auftreten, da das röntgenopake Material häufig nur eine geringe Ausdehnung in der Ebene der Bilderfassung und senkrecht dazu aufweist. Kleine Flächen, beispielsweise in der Größenordnung von derzeit 200 µm x 200 µm in der Erfassungsebene, ergeben bei derzeit verwendeten Messeinrichtungen etwa einen Pixel mit einem Messsignal. Diese Signale werden, wenn sie vereinzelt auftreten, bei der maschinellen Bilderstellung nach Abschluss der Messung häufig wieder eliminiert, da derartige Bildbestandteile häufig als Fehlmessung klassifiziert werden. Daher muss eine möglichst große Ausdehnung des röntgenopaken Materials in der Erfassungsebene erreicht werden, so dass mehrere, nebeneinander liegende Pixel Messsignale zeigen. Außerdem ist eine große Ausdehnung des Röntgenmarkers in eine Richtung senkrecht zur Erfassungsebene deshalb von Vorteil, weil der absorbierte Anteil der Röntgenstrahlen proportional zur Dicke des durchstrahlten Materials ist. Da der Stent beliebig im Körper und somit zur Erfassungsebene orientiert sein kann, ist es folglich wünschenswert, dass das von dem Röntgenmarker ausgefüllte Volumen möglichst groß ist, um Messfehler bei der Stent-Positionsbestimmung zu vermeiden.

Die Röntgenmarker bei oben genannten, bekannten Stents weisen nur ein kleines Volumen auf. Bei der Anordnung von röntgenopakem Material in Ausnehmungen ist das Volumen des Materials durch die inneren Abmessungen der Ausdehnung begrenzt. Bei einer Beschichtung des Stent-Grundgitters mit röntgenopakem Material ist die Dicke dieser Schichten klein, da bei größeren Schichtdicken die Beeinflussung der Strömung der in den Gefäßen fließenden Flüssigkeiten, beispielsweise Blut, durch die beschichteten Stentabschnitte zu groß wird und hierdurch eine Restenose begünstigt werden kann.

Bekannt ist außerdem, Funktionselemente an Stents vorzusehen, die Medikamente aufweisen, beispielsweise mit antiinflammatorischer oder antiproliferativer Wirkung. Auch im Hinblick auf diese Funktionselemente ist es wünschenswert, dass das Volumen der Funktionselemente möglichst groß gewählt ist, so dass ein größeres Wirkstoffvolumen aufgenommen und hierdurch die Wirkdauer der Medikamente verlängert werden kann.

Bei Stents, deren Grundgitter aus einer absorbierbaren Magnesiumlegierung besteht, ergibt sich bei der Anordnung von Funktionselementen, beispielsweise bei Markerelementen aus Gold oder Silber, an dem Stent-Grundgitter das zusätzliche Problem, dass an dem Kontaktbereich zwischen beiden Materialien eine Kontaktkorrosion auftritt. Diese führt zur Zerstörung des Stents bzw. zur Abtrennung des Funktionselements von der Stentstruktur.

Die Aufgabe der vorliegenden Erfindung ist es daher, eine Endoprothese zu schaffen, bei der das Funktionselement ein großes Volumen aufweist. Gleichzeitig sollte gewährleistet werden, dass das Funktionselement möglichst geringen mechanischen Belastungen ausgesetzt ist, so dass ein Defekt des Stents im Bereich des Funktionselements, insbesondere eine Abtrennung des Funktionselements vom Grundgitter des Stents verhindert wird. Die Aufgabe bestand ferner darin, ein kostengünstiges Verfahren zur Herstellung einer derartigen Endoprothese anzugeben.

Diese Aufgabe wird durch eine Endoprothese gelöst, deren Trägerstruktur an einem ersten, im Wesentlichen fingerförmigen Ende, d.h. einseitig, an dem Grundgitter angeordnet ist und von diesem im Wesentlichen fortsatzartig wegragt, wobei das Funktionselement an einem von dem Grundgitter wegragenden Bereich der Trägerstruktur angeordnet ist und diesen Bereich mindestens teilweise umgibt.

Bei dem erfindungsgemäßen Stent wird das Funktionselement lediglich in einem punktförmigen Bereich, nämlich an dem ersten, fingerförmigen Ende der Trägerstruktur an dem Grundgitter angeordnet und befestigt, so dass diese sich flexibel an und mit dem Grundgitter bewegen kann, so dass beispielsweise bei der Dilatation des Grundgitters keine plastische Verformung auftritt und die vom Grundgitter auf die Trägerstruktur übertragene Spannungen abgebaut werden können. Außerdem umgibt das Funktionselement die Trägerstruktur in einem von dem Grundgitter wegragenden Bereich der Trägerstruktur, die in dem Bereich vorzugsweise einen geringeren Durchmesser als die Strukturen des Grundgitters aufweist, so dass das Volumen des Funktionselements größer als bei den bekannten Lösungen ist.

In einer bevorzugten Ausführungsform umschließt oder umgibt das Funktionselement das vom Grundgitter wegragende, zweite Ende der Trägerstruktur vollständig. Hierdurch wird ein besonders großes Volumen des Funktionselements und zusätzlich eine gute Verankerung und Befestigung des Funktionselements an der Trägerstruktur erreicht.

Besonders im Hinblick auf das Volumen bilden eine Tropfen-, eine Scheiben- oder eine Kugelform vorteilhafte Formen des Funktionselements. Jede andere beliebige, dem Fachmann bekannte und herstellbare geometrische Form wie Ellipsoidform, Quader- oder Würfelform bzw. Sternform kann alternativ vorgesehen werden. Diese Formen lassen sich kostengünstig herstellen.

In vorteilhafter Weise besteht das Funktionselement mindestens teilweise aus einem röntgenopaken Material, so dass die Position des Stents im Körper einfach bestimmt werden kann. Für das röntgenopake Material werden bevorzugt eines oder mehrere der Elemente aus der Gruppe Gold, Platin, Silber, Wolfram, Iod, Tantal, Yttrium, Niob, Molybdän, Ruthenium, Rhodium, Barium, Lanthan, Cer, Praseodym, Neodym, Samarium, Europium, Gadolinium, Terbium, Dysprosium, Holmium, Erbium, Thulium, Ytterbium, Lutetium, Hafnium, Rhenium, Osmium und Bismut und/oder eine oder mehrere der röntgenopaken Verbindungen aus diesen Elementen und/oder Bariumsulfat, Bismuttrioxid, Bromin, Iodin, Iodid, Titanoxid und Zirkonoxid verwendet.

Besonders bevorzugt besteht das Funktionselement aus einer Markerlegierung, welche aus DE 103 61 942 bekannt ist. Dann besteht das Funktionselement aus einer biodegradierbaren Basiskomponente oder Basislegierung, insbesondere aus der Gruppe der Elemente Magnesium, Eisen, Wolfram und/oder Zink. Weiterhin enthält das Funktionselement eines oder mehrere der oben genannten röntgenopaken Elemente und/oder eine oder mehrere röntgenopake Verbindungen aus diesen Elementen.

Besonders bevorzugt ist eine Zusammensetzung MgₓYb_{y} mit x = 10 - 60 Atomprozent und y = 40 - 90 Atomprozent, wobei x und y zusammen und einschließlich herstellungsbedingter Verunreinigungen 100 Atomprozent ergeben. Weiter besonders bevorzugt ist die Zusammensetzung Mg_{31.5}Yb_{68.5} des Funktionselement-Materials.

Alternativ oder zusätzlich kann mindestens ein Funktionselement der Endoprothese mit einer pharmazeutisch aktiven Substanz versehen sein.

Eine "pharmazeutisch aktive Substanz" im Sinne der Erfindung ist ein pflanzlicher, tierischer oder synthetisierter Wirkstoff, der in geeigneter Dosierung als Therapeutika zur Beeinflussung von Zuständen oder Funktionen des Körpers, als Ersatz für natürlich vom menschlichen oder tierischen Körper erzeugte Wirkstoffe sowie zur Beseitigung oder zum Unschädlichmachen von Krankheitserregern oder Körperfremdstoffen Einsatz findet. Die Freisetzung der Substanz in der Endoprothesenumgebung hat einen positiven Effekt auf den Heilungsverlauf bzw. wirkt pathologischen Veränderungen des Gewebes infolge des chirurgischen Eingriffes entgegen.

Erfindungsgemäß weisen die pharmazeutisch aktiven Substanzen eine antiinflammatorische und/oder antiproliferative und/oder spasmolytische und/oder endothelbildende Wirkung auf, wodurch Restenosen, Entzündungen oder Gefäßspasmen vermieden werden können.

Bevorzugte Wirkstoffe, insbesondere zur Therapie oder Prophylaxe der In-Stent-Restenose, die zur Inkorporierung in eine Polymermatrix eines erfindungsgemäßen Implantats besonders geeignet sind, werden ausgewählt aus der Gruppe bestehend aus:
- Lipidregulatoren (Fibrate),
- Immunsuppressiva,
- Vasodilatatoren (Sartane),
- Calciumkanalblocker,
- Calcineurininhibitoren (Tacrolimus),
- Antiflogistika (Cortison, Dichlofenac),
- Antiinflammatorica (Imidazole),
- Antiallergika,
- Oligonucleotide (dODN),
- Östrogene (Genistein),
- Endothelbildner (Fibrin),
- Steroide,
- Proteine / Peptide,
- Proliferationshemmer
- Analgetika
- Antirheumatika

Ferner kann das Funktionselement Polymere oder körpereigene Stoffe enthalten, die als Matrixmaterial die genannten röntgenopaken Materialien und/oder die genannten pharmazeutisch aktiven Substanzen aufnehmen. Bevorzugt kommen als derartige Polymere resorbierbare (d.h. bioresorbierbare oder degradierbare) Polymere oder nicht resorbierbare (d.h. permanente oder nicht degradierbare) Polymere zum Einsatz, besonders bevorzugt Collagene oder Cholesterole.

Bevorzugte Polymere für die Polymermatrix des erfindungsgemäßen Implantats werden ausgewählt aus der Gruppe:
- nichtresorbierbarer / permanenter Polymere wie beispielsweise: Polypropylen, Polyethylen, Polyvinylchlorid, Polyacrylate (Polyethyl- und Polymethylacrylate, Polymethylmetacrylat, Polymethyl-co-ethyl-acrylat, Ethylen/Ethylacrylat) Polytetrafluorethylen (Ethylen/Chlortrifluorethylen Copolymer, E-thylen/Tetrafluorethylen Copolymer), Polyamide (Polyamidimid, PA-11, -12, -46, - 66), Polyetherimid, Polyethersulfon, Poly(iso)butylen, Polyvinylchlorid, -fluorid, Polyvinylalkohol, Polyurethan, Polybuthylenterephthalat, Silikone, Polyphosphazen, Polymerschäume (aus z.B. Carbonaten, Styrolen), sowie die Copolymere und Blends der aufgezählten Klassen, bzw. der Klasse der Thermoplaste und der Elastomere im Allgemeinen
- resorbierbarer / bioresorbierbarer / degradierbarer Polymere wie beispielsweise: Poly-dioxanon, Polyglycolid Polycaprolacton, Polylactide [Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(I-Lactid-co-trimethylen carbonat)], Triblockcopolymere, Polysaccharide [Chitosan, Levan, Hyaluronsäure, Heparin, Dextran, Cellulose etc.], Polyhydroxyvalerat, Ethylvinylacetat, Polyethylenoxid, Polyphosphorylcholin, Fibrin, Albumin, Polyhydroxybuttersäure (ataktisch, isotaktisch, syndiotaktisch sowie deren Blends), etc.
   Besonders bevorzugt sind Polylactide [Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(L-Lactid-co-trimethylen carbonat)]

In einem bevorzugten Ausführungsbeispiel ist die Trägerstruktur besonders einfach und kostengünstig herstellbar, wenn diese im Wesentlichen fingerförmig ausgebildet ist. In einem weiteren bevorzugten Ausführungsbeispiel weist die Trägerstruktur einen ersten Finger und an ihrem zweiten Ende mindestens einen zweiten, von dem ersten Finger abzweigenden Finger auf. Diese Trägerstruktur schafft einerseits die im Wesentlichen punktförmige, flexible Anbindung des Funktionselements an dem Grundgitter und andererseits, durch die Verzweigung an dem zweiten Ende, eine Struktur, an der das Material des Funktionselements gut befestigt werden kann. Die Trägerstruktur weist eine große Oberfläche auf, die zur Verankerung des Funktionselements dient. Diese erfindungsgemäße Lösung kann weiter verbessert werden, wenn die Trägerstruktur an ihrem zweiten Ende eine Vielzahl fraktaler Verzweigungen aufweist. Ebenfalls eine vorteilhafte große Oberfläche zur Verankerung des Funktionselements bietet die Trägerstruktur, wenn diese an ihrem zweiten Ende baumförmige Verästelungen ausbildet.

Das von dem Funktionselement eingenommene Volumen kann noch vergrößert werden, wenn die Trägerstruktur zumindest in dem Bereich, in dem das Funktionselement an der Trägerstruktur angeordnet ist, in radialer Richtung zu dem hohlzylinderförmigen Grundgitter eine kleinere Ausdehnung als die Grundgitter-Struktur aufweist.

In einem weiteren bevorzugten Ausführungsbeispiel besteht die Trägerstruktur mindestens teilweise aus einem elektrisch isolierenden Material, insbesondere Kunststoff und/oder Keramik. Hierbei ist das elektrisch isolierende Material so angeordnet, dass das Grundgitter und das Funktionselement elektrisch voneinander isoliert werden, so dass eine Kontaktkorrosion verhindert wird.

Zur Behebung des Problems der Kontaktkorrosion ist bei einem weiteren bevorzugten Ausführungsbeispiel der Erfindung vorgesehen, dass die Trägerstruktur, die beispielsweise kostengünstig aus dem Material des Grundgitters bei der Herstellung des Grundgitters ausgebildet sein kann, mit einem elektrisch isolierenden Material, beispielsweise mit einem Kunststoff und/oder einer Keramik, besonders bevorzugt mit einer derartigen isolierenden Beschichtung insbesondere dort versehen wird, wo das Funktionselement an der Trägerstruktur befestigt ist. Durch die Beschichtung mit dem elektrisch isolierenden Material wird ein Kontakt zwischen der unedlen Magnesiumlegierung und dem edleren röntgenopaken Material verhindert.

Nach einem besonders bevorzugten Ausführungsbeispiel sind an der Endoprothese eine Vielzahl von Trägerstrukturen mit Funktionselementen aufweisend eine pharmazeutisch aktive Substanz vorgesehen, die gleichmäßig über die gesamte Wand der Gitterstruktur verteilt angeordnet sind. Hierdurch wird eine räumlich besonders gut verteilte Freisetzung der pharmazeutisch aktiven Substanz erreicht.

Alternativ oder zusätzlich ist eine Vielzahl von Trägerstrukturen mit Funktionselementen aufweisend röntgenopakes Material an dem distalen und/oder proximalen Ende der Endoprothese, vorzugsweise auf jeweils einer Umfangslinie angeordnet. Eine Positionierung des Stents ist hierdurch besonders einfach möglich, da die Anbringung der Funktionselemente an einem im Hinblick auf einen gut definierten Abschnitt des Stents erfolgt.

Die Aufgabe wird außerdem mit einem Verfahren zur Herstellung einer Endoprothese gelöst, bei dem das Grundgitter zusammen mit der mindestens einen Trägerstruktur aus einem Hohlzylinder hergestellt wird. Dies führt dazu, dass die Trägerstrukturen größeren mechanischen Belastungen ausgesetzt werden können. Die gemeinsame Herstellung von Grundgitter- und Trägerstrukturen ist zudem kostengünstig.

Hierbei werden die zwei- oder dreidimensionalen Grundgitter- und Trägerstrukturen mittels Laserstrahl- oder Wasserstrahlschneiden oder mittels chemischer oder elektrochemischer Ätzverfahren mit und ohne Anwendung lithographischer Techniken hergestellt. Anschließend können in einer bevorzugten Ausführungsform mechanische Umformverfahren (z.B. Pressen), beispielsweise zur Verringerung der Ausdehnung der Trägerstruktur in radialer Richtung gegenüber der des Grundgitters, und/oder Biegen, vorzugsweise unter Beibehaltung der Ausdehnung der Trägerstruktur verglichen mit der des Grundgitters, durchgeführt werden.

Alternativ wird die Aufgabe außerdem mit einem Verfahren zur Herstellung einer Endoprothese gelöst, bei dem die mindestens eine Trägerstruktur separat vom Grundgitter gefertigt wird und anschließend am Grundgitter befestigt wird. Vorzugsweise wird die Trägerstruktur mit dem Funktionselement anhand eines weiter unten beschriebenen Verfahrens versehen, bevor die Trägerstruktur an dem Grundgitter befestigt wird. Hierbei werden die zwei- oder dreidimensionalen Trägerstrukturen zunächst mittels Laserstrahl- oder Wasserstrahlschneiden oder mittels chemischer oder elektrochemischer Ätzverfahren mit oder ohne Einsatz lithographischer Techniken oder mittels Stanzen vorzugsweise aus einem plattenförmigen Ausgangskörper gefertigt. In einem bevorzugten Ausführungsbeispiel können anschließend die Trägerstrukturen mittels mechanischer Umformverfahren, beispielsweise Pressen oder Biegen, weiterbearbeitet und verfeinert werden. Ggf. kann die Trägerstruktur einem Sinterschritt unterzogen werden.

Die vorgefertigte Trägerstruktur kann dann an dem Grundgitter mittels Schweißen, Löten oder Kleben befestigt werden. Weiterhin kann die Trägerstruktur auch an ihrem ersten fingerförmigen Ende als Passungsstift ausgebildet sein, welcher in eine Passung am Grundgitter durch eine Presspassung befestigt ist. Die Trägerstruktur kann auch an das Grundgitter angeklippst sein.

Die Funktionselemente können mittels Schweißen, Löten, Kleben oder Pressen an der jeweiligen Trägerstruktur, die separat oder gemeinsam mit dem Grundgitter hergestellt wurde, befestigt werden.

Ebenso ist es möglich, das Funktionselement an der jeweiligen Trägerstruktur mittels Sprühen, Tauchen, Tunken oder anderer bekannter Beschichtungsverfahren zu befestigen. Ferner kann eine mechanisch stabile Beschichtung vorgesehen werden, welche bei einem als Depot für pharmazeutisch aktive Substanzen ausgebildeten Funktionselement zwischen der Trägerstruktur und dem Funktionselement liegt oder welche bei einem als Markerelement ausgebildeten Funktionselement die Oberfläche des Funktionselements ausbildet.

Eine weitere Möglichkeit zur Befestigung der Funktionselemente besteht darin, das röntgenopake Material bzw. die pharmazeutisch aktive Substanz in eine Matrix aus einem Kohlenstoffpolymer oder einem anderen Kunststoff, vorzugsweise aus einem oben angegebenen degradierbaren Polymer, einzubetten, ein Tropfen hiervon an der jeweiligen Trägerstruktur anzubringen und das Polymer anschließend beispielsweise durch Polymerisation, vorzugsweise unter Anwendung einer Reagenz zum Initiieren der Polymerisation, oder durch Härten, vorzugsweise durch IR-Aushärten, oder durch Trocknen, vorzugsweise durch Trocknen mittels Laser, oder mittels eines Pyrolyseprozesses oder durch andere, dem Fachmann bekannte gängige Verfahren zu verfestigen. Zum Einbringen des röntgenopaken Materials oder des Wirkstoffs in die Matrix liegen diese vorzugsweise in einer Korngröße von mehreren Mikrometern oder kleiner, besonders bevorzugt als nanokristallines Material vor. Für das Anbringen der Tropfen an der Trägerstruktur kann vorzugsweise eine entsprechend geformte Gießform verwendet werden. Ähnlich kann auch vorgegangen werden, wenn als Matrixmaterial ein keramisches Material verwendet wird.

In einem weiteren Ausführungsbeispiel kann das röntgenopake Materialen und/oder die pharmazeutisch aktive Substanz mittels eines beispielsweise in DE 100 64 596 A1 beschriebenen Verfahrens an der jeweiligen Trägerstruktur befestigt werden. Hierfür sind ebenfalls Formen vorgesehen, die das zu verfestigende Material so anordnen, dass die Funktionselemente nach Abschluss des Herstellungsverfahrens an der jeweiligen Trägerstruktur befestigt sind. Der Inhalt dieser Druckschrift wird hiermit in diese Beschreibung aufgenommen.

Bei der Herstellung der Trägerstrukturen, ggf. mit den Funktionselementen, zusammen mit dem Grundgitter aus einem Hohlzylinder kann dieser mindestens bereichsweise auch als Materialverbund mindestens zweier verschiedener Materialien ausgebildet sein. In einem bevorzugten Ausführungsbeispiel kann beispielsweise ein Hohlzylinder aus einer biodegradierbaren Magnesiumlegierung vorgesehen sein, an den z.B. mittels Reibschweißen ein im Durchmesser kleinerer Hohlzylinder bestehend aus dem Material der Trägerstruktur (und/oder ggf. einer Markerlegierung) angebracht wurde. Die Bearbeitung mittels obiger Verfahren kann dann derart erfolgen, dass nur an den Stellen, an denen die Trägerstrukturen bzw. Funktionselemente entstehen sollen, die entsprechende Schicht "stehen gelassen", d.h. nicht entfernt wird.

Weitere Ziele, Merkmale, Vorteile und Anwendungsmöglichkeiten der Erfindung ergeben sich aus der nachfolgenden Beschreibung von Ausführungsbeispielen anhand von Zeichnungen. Dabei bilden alle beschriebenen und/oder bildlich dargestellten Merkmale für sich oder in beliebiger Kombination den Gegenstand der Erfindung, unabhängig von ihrer Zusammenfassung in einzelnen Ansprüche oder deren Rückbeziehung.

Es zeigen:
- Fig. 1 a bis 6: schematisch jeweils einen Ausschnitt aus einem proximalen oder distalen Endabschnitt verschiedener Ausführungsformen erfindungsgemäßer Stents in jeweils einer Ansicht von der Seite mit Teilschnitten durch das Stent-Grundgitter im Bereich der Trägerstruktur, durch die Trägerstruktur und durch das Funktionselement,
- Fig.7: schematisch einen Längsschnitt durch einen Grundgitterabschnitt einer weiteren Ausführungsform eines erfindungsgemäßen Stents mit einem Längsschnitt durch eine Trägerstruktur und ein Funktionselement und
- Fig. 8: schematisch einen Ausschnitt aus der Stentwand einer weiteren Ausführungsform eines erfindungsgemäßen Stents in einer Ansicht von der Seite.

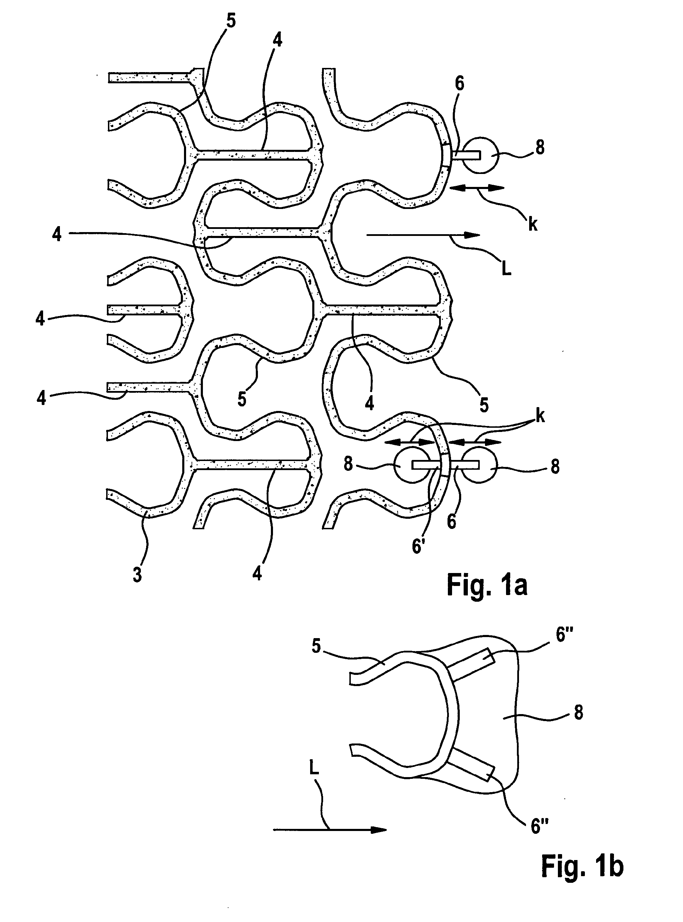

Der in Fig. 1 a gezeigte Ausschnitt eines Endabschnitts eines ersten Ausführungsbeispiels eines erfindungsgemäßen Stents weist ein Grundgitter 3 mit in Längsrichtung L verlaufenden Stegen 4 und zick-zack- oder mäanderförmig geformten Stegen 5 auf. Die zick-zack- oder mäanderförmig gefalteten Stege 5 sind mit den in Längsrichtung L verlaufenden Stegen 4 verbunden und bilden gemeinsam das Gittergerüst des Stents, das insgesamt als in Längsrichtung L verlaufende Röhre geformt ist.

Das Grundgitter des Stents besteht vorzugsweise aus einem metallischen Material aus einem oder mehreren Metallen aus der Gruppe Eisen, Magnesium, Nickel, Wolfram, Titan, Zirkonium, Niob, Tantal, Zink oder Silizium und ggf. einer zweiten Komponente aus einem oder mehreren Metallen aus der Gruppe Lithium, Natrium, Kalium, Kalzium, Mangan, Eisen oder Wolfram, vorzugsweise aus einer Zink-Kalziumlegierung. In einem weiteren Ausführungsbeispiel besteht das Grundgitter 3 aus einem Formgedächtnis-Material aus einem oder mehreren Materialien aus der Gruppe bestehend aus Nickel-Titan-Legierungen und Kupfer-Zink-Aluminium-Legierungen, vorzugsweise besteht das Grundgitter 3 aus Nitinol. In einem weiteren bevorzugten Ausführungsbeispiel besteht das Grundgitter 3 des Stents aus Edelstahl, weiter bevorzugt aus der Legierung 316L, vorzugsweise aus einem Cr-Ni-Fe-Stahl oder einem Co-Cr-Stahl. Ferner kann das Grundgitter des Stents mindestens teilweise aus Kunststoff und/oder einer Keramik bestehen.

In einem weiteren Ausführungsbeispiel besteht das Grundgitter aus einer absorbierbaren Magnesiumlegierung der folgenden Zusammensetzung:
- Seltene Erden 2,0 bis 30,0 Gew.-%,
- Yttrium 0,0 bis 20,0 Gew.-%,
- Zirkonium 0,3 bis 5 Gew.-%,
- Rest 0 bis 10,0 Gew.-% (ggf. z.B. Neodym),
wobei Magnesium (mindestens 60,0 Gew.-%) den auf 100 Gew.-% verbleibenden Gewichtsanteil an der Legierung einnimmt.

Das Stent-Grundgitter 3 wird dadurch hergestellt, dass zunächst ein Grundkörper in Form eines Hohlzylinders (Röhre) bereitgestellt wird, aus dem die Struktur des Grundgitters z.B. mittels einer Schneidetechnik, vorzugsweise mittels Laser- oder Wasserstrahlschneiden, herausgeschnitten oder mittels chemischer oder elektrochemischer Ätzverfahren mit oder ohne Anwendung lithographischer Techniken hergestellt wird. Anschließend kann die Oberfläche des Stent-Grundgitters noch bearbeitet, insbesondere veredelt, geglättet und/oder poliert werden.

An dem in Fig. 1 a dargestellten Ausführungsbeispiel eines erfindungsgemäßen Stents sind an den am weitesten in Richtung des proximalen oder distalen Endes des Stents angeordneten zick-zack- oder mäanderförmigen Stegen 5 fingerförmige Trägerstrukturen 6 befestigt. Entlang einer Richtung k, welche die Erstreckungsrichtung der Längsachse der Trägerstruktur angibt und die in diesem Fall parallel zur Längsrichtung L des Stents verläuft, weisen die Trägerstrukturen 6 ihre größte Ausdehnung auf. Die Trägerstrukturen 6 können beispielsweise eine Zylinder- oder Quaderform aufweisen, so dass der Querschnitt senkrecht zur Richtung k im Wesentlichen rechteckig oder rund ausgebildet ist. Die Trägerstrukturen 6 sind mit ihrem ersten fingerförmigen Ende entlang der Richtung k an dem Steg 5 befestigt und ragen in Längsrichtung L von dem Grundgitter 3 des Stents derart weg, dass das zweite, wegragende Ende der Trägerstruktur 6 vom Grundgitter 3 weg gerichtet ist.

Eine weitere, fingerförmige Trägerstruktur 6' ist analog zu der Trägerstruktur 6 mit dem ersten fingerförmigen Ende an dem zick-zack- oder mäanderförmigen Steg 5 angeordnet. Diese Trägerstruktur 6' ragt in Längsrichtung L des Stents von dem Steg 5 derart weg, dass das zweite Ende in Längsrichtung k der Trägerstruktur 6' in Richtung des Grundgitters zeigt. Hierdurch ist diese Trägerstruktur 6' im Bereich der Stent-Wand durch den zick-zack- oder mäanderförmigen Steg 5 "eingerahmt", d.h. die mäandernden Bereiche des Stegs 5 umgeben die Trägerstruktur 6' an drei Seiten im Bereich der Stent-Wand. Aufgrund der Befestigung der Trägerstrukturen 6, 6' an ihrem ersten fingerförmigen Ende an dem Grundgitter 3 kann sich die jeweilige Trägerstruktur 6, 6' flexibel den Bewegungen des zick-zack- oder mäanderförmigen Stegs 5 anpassen.

In einer besonders bevorzugten Ausführungsform in Fig. 1b ragen zwei oder mehrere Trägerstrukturen 6" im Wesentlichen in Längsrichtung L des Stents von dem Steg 5 weg.

An den Trägerstrukturen 6, 6' ist jeweils an dem zweiten Ende, das dem ersten Ende in Längsrichtung der Trägerstruktur 6, 6' gegenüberliegt, ein Funktionselement 8 angeordnet, das eine Kugelform aufweist. Hierbei umgibt das Funktionselement 8 das zweite Ende der Trägerstruktur 6, 6' vollständig. In einem weiteren Ausführungsbeispiel kann das Funktionselement 8 auch eine Scheibenform aufweisen, wobei sich der kreisförmige Querschnitt im Wesentlichen in einer Ebene erstreckt, die tangential zur Mantelfläche des zylinderförmigen Stents verläuft (Tangentialebene). Das kugelförmige Funktionselement 8 umgibt das vom Grundgitter wegragende Ende der Trägerstruktur 6, 6' vollständig. Hierdurch wird eine möglichst große Ausdehnung des Funktionselements in Richtung der Tangentialebene erreicht.

Das Funktionselement 8 kann röntgenopakes Material, bevorzugt eines oder mehrerer oben angegebener röntgenopaker Elemente und/oder eines oder mehrerer oben angegebener röntgenopaker Verbindungen enthalten. Beispiele für das Material des Funktionselements sind ebenfalls oben gegeben.

Zusätzlich oder alternativ kann das Funktionselement 8 pharmazeutisch aktive Substanzen mit antiinflammatorischer, antiproliferativer und/oder spasmolytischer Wirkung und beispielsweise aus der oben genannten Wirkstoffgruppe aufweisen, welche mit Hilfe einer vorzugsweise polymeren Trägermatrix mit der Trägerstruktur 6, 6' verbunden sein können. Nach Implantation des Stents können diese Wirkstoffe in das Körpergewebe eluieren und ihre antiinflammatorische, antiproliferative und/oder spasmolytische Wirkung entfalten. Dabei ist in einer besonders bevorzugten Ausführungsform vorgesehen, dass die als Wirkstoffdepot ausgebildeten Funktionselemente gleichmäßig über die gesamte Wand der Gitterstruktur verteilt angeordnet sind.

In einem bevorzugten Ausführungsbeispiel sind die Trägerstrukturen 6, 6' aus einem isolierenden Material, beispielsweise aus einer Keramik oder einem Kunststoff gefertigt.

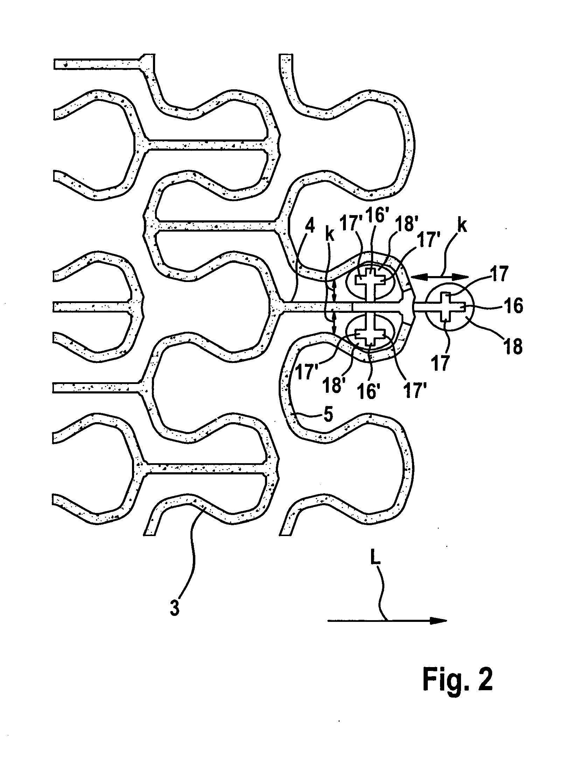

In dem in Fig. 2 dargestellten Ausführungsbeispiel eines erfindungsgemäßen Stents sind Trägerstrukturen 16 dargestellt, welche an ihrem ersten Ende fingerförmig ausgebildet sind und mit diesem Ende an dem Grundgitter 3 des Stents befestigt sind. An dem zweiten Ende weisen die Trägerstrukturen 16 zwei jeweils seitlich, d.h. senkrecht zur Richtung k, wegragende Finger 17 auf. Die Trägerstruktur 16 bildet somit an ihrem zweiten Ende eine Kreuzform aus. Die Trägerstruktur 16 ist mit dem zick-zack- oder mäanderförmigen Steg 5 verbunden. Eine weitere, analog aufgebaute Trägerstruktur 16' ist mit ihrem ersten, fingerförmigen Ende mit dem in Längsrichtung L verlaufenden Steg 4 des Grundgitters 3 verbunden und ragt von diesem in der Tangentialebene im Wesentlichen senkrecht zur Längsrichtung L weg.

Im Falle der Trägerstruktur 16 ist das Funktionselement 18, das an dem Ende der Trägerstruktur 16 angeordnet ist, das von dem Grundgitter 3 des Stents wegragt, im Wesentlichen kugel- oder scheibenförmig analog zu den in Fig. 1 a oder 1b dargestellten Ausführungsbeispielen gestaltet. Die an den Trägerstrukturen 16' vorgesehenen Funktionselemente 18' sind im Wesentlichen tropfenförmig gestaltet und umgeben jeweils das zweite Ende der Trägerstruktur 16' einschließlich der seitlich, senkrecht zur Richtung k wegragenden Finger 17' vollständig.

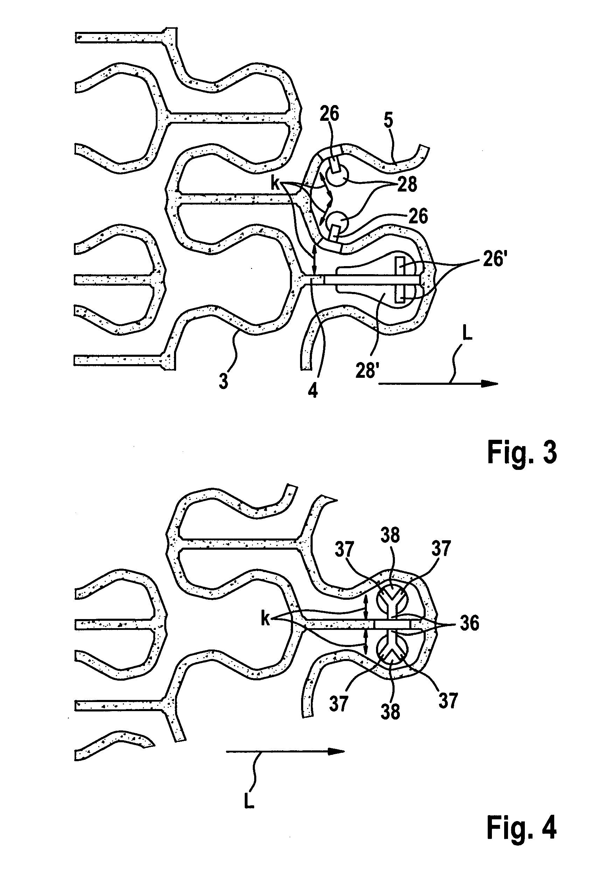

Die in Fig. 3 dargestellten Trägerstrukturen 26 entsprechen den fingerförmigen Trägerstrukturen 6, 6' aus Fig. 1 a oder 1b. Diese sind an den am weitesten in Richtung Ende des Stents liegenden zick-zack- oder mäanderförmigen Stegen 5 in den Bereichen dieser Stege 5 angeordnet, die in Richtung des Grundgitters 3 nach innen gekrümmt sind (konkaver Abschnitt). Die zick-zack- oder mäanderförmigen Stege 5 umgeben somit die Trägerstrukturen 26 und die an dem zweiten Ende der Trägerstruktur 26 befestigten Funktionselemente 28 im Bereich der Stent-Wand an drei Seiten. Hierbei weisen die Funktionselemente 28 eine Tropfenform auf.

Ein weiteres, anhand von Fig. 3 dargestelltes Ausführungsbeispiel weist zwei fingerförmige Trägerstrukturen 26' auf, die mit ihrem einen Ende an einem in Längsrichtung L verlaufenden Steg 4 in der Tangentialebene gegenüberliegend angeordnet sind. Die Längsrichtung k der Trägerstrukturen 26' verläuft somit senkrecht zur Längsrichtung L des Stents. Das Funktionselement 28' erstreckt sich in einer Tropfenform so um die gegenüberliegenden Trägerstrukturen 26', dass das Funktionselement 28' beide Trägerstrukturen 26' vollständig umschließt und zusätzlich den nächstliegenden Bereich des in Längsrichtung verlaufenden Stegs 4, an dem die Trägerstrukturen 26' befestigt sind. Hierdurch wird eine besonders große Ausdehnung des Funktionselements 28' im Wesentlichen in der Längsrichtung L des Stents erreicht.

Analog zu dem Funktionselement 28' kann auch das tropfenförmige Funktionselement 28 in einem weiteren, nicht dargestellten Ausführungsbeispiel so weit ausgedehnt werden, dass es bis zu dem jeweiligen Steg 5 reicht.

Anhand von Fig. 4 ist ein weiteres Ausführungsbeispiel eines erfindungsgemäßen Stents mit einer Trägerstruktur 36 dargestellt, wobei die Trägerstruktur 36 in einem Längsschnitt im Wesentlichen eine Y-Form aufweist. Die Trägerstruktur 36 ist mit ihrem ersten fingerförmigen Ende mit einem in Längsrichtung L verlaufenden Steg 4 verbunden. An ihrem zweiten Ende weist die Trägerstruktur 36 zwei in einem spitzen Winkel voneinander weg stehende Finger 37 auf. Das Funktionselement, das kugel- oder scheibenförmig ausgebildet ist, umgibt das vom Grundgitter 3 wegragende zweite Ende der Trägerstruktur 36 mit den Fingern 37 vollständig.

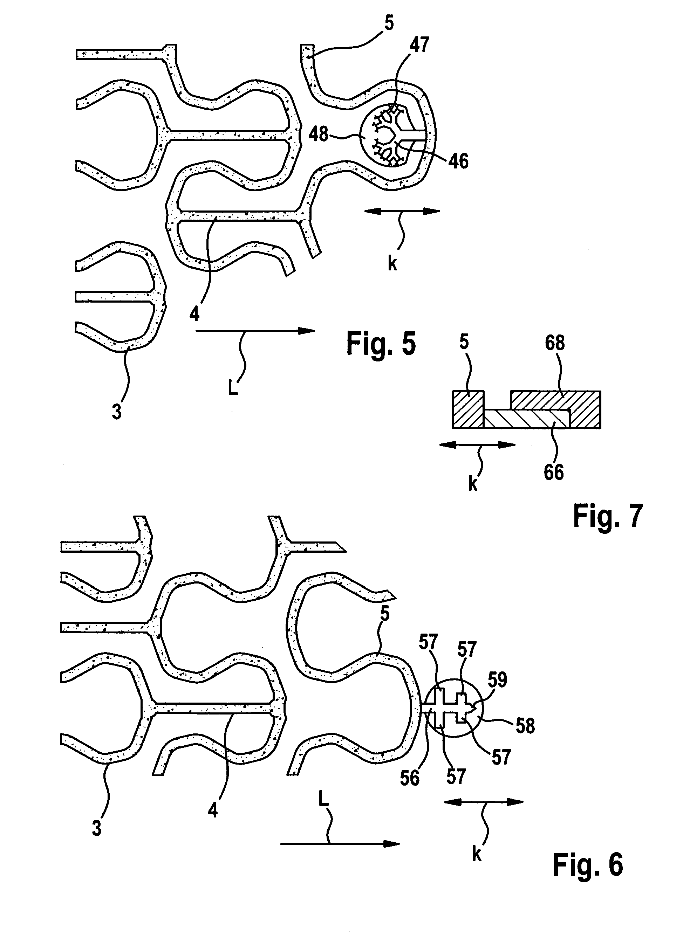

Das anhand von Fig. 5 dargestellte Ausführungsbeispiel eines erfindungsgemäßen Stents weist eine Trägerstruktur 46 auf, die an dem ersten Ende, das mit dem Steg 5 des Stents verbunden ist, fingerförmig ausgebildet ist. An dem zweiten, vom Grundgitter 3 wegragenden Ende weist die Trägerstruktur 46 fraktale Verästelungen 47 auf. Die fraktalen Verästelungen sind dabei vorzugsweise in der Ebene der Stent-Wand vorgesehen. In weiteren Ausführungsbeispielen können auch in einer radial in Bezug auf den Stent verlaufenden Ebene Verästelungen vorgesehen sein. Das Funktionselement 48 umgibt das zweite Ende der Trägerstruktur 46 mit den fraktalen Verästelungen 47 in einer Tropfenform vollständig. Hierdurch wird eine besonders feste Verankerung des Funktionselements 48 an der Trägerstruktur 46 erreicht.

In einem weiteren, anhand von Fig. 6 dargestellten Ausführungsbeispiel eines erfindungsgemäßen Stents weist die Trägerstruktur 56 an ihrem zweiten Ende mehrere seitlich angeordnete Finger 57 auf, deren Länge in Längsrichtung k in Richtung zweites Ende der Trägerstruktur 56 geringer wird, so dass im Wesentlichen eine Baumstruktur ausgebildet wird. Hierbei umgeben die Finger 57 einen den Stamm darstellenden Bereich 59 der Trägerstruktur 56 vorzugsweise um den gesamten Umfang herum. Analog zu den vorangegangenen Ausführungsbeispielen umgibt das Funktionselement 58 das zweite Ende der Trägerstruktur 56 mit der Baumstruktur 57, 59 vollständig.

In weiteren, nicht dargestellten Ausführungsbeispielen kann die Trägerstruktur an ihrem zweiten Ende auch andere als die oben dargestellten Formen aufweisen, die eine gute Befestigung des an diesem Ende angeordneten, die Trägerstruktur mindestens teilweise umschließenden Funktionselements gewährleisten.

Das in Fig. 7 gezeigte Ausführungsbeispiel eines erfindungsgemäßen Stents weist eine fingerförmige Trägerstruktur 66 analog zu der in Fig. 1 a oder 1b dargestellten Struktur 6 auf, die in radialer Richtung bezogen auf den Stent eine geringere Dicke als der Stentsteg 5 hat. Das im Wesentlichen scheibenförmige Funktionselement 68 umgibt die Trägerstruktur 66 an dem zweiten Ende, wobei das Funktionselement 68 lediglich an der Oberseite der Trägerstruktur 66 angeordnet ist. Hierdurch wird erreicht, dass die Gesamtdicke des Elements aus Trägerstruktur 66 und Funktionselement 68 vergleichbar ist mit der Dicke der Stege 4, 5 des Stents, so dass sowohl ein großes Volumen des Funktionselements 68 als auch eine geringe Beeinflussung der Strömung der in dem Gefäß fließenden Flüssigkeiten erreicht wird.

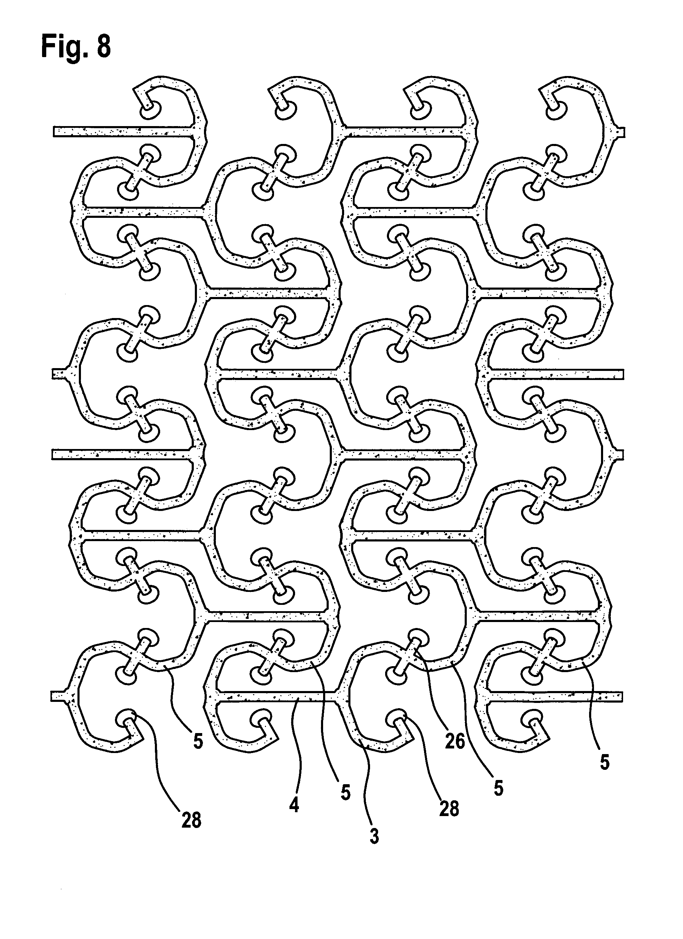

Insgesamt kann der Stent über seine gesamte Länge verteilt die anhand der Fig. 1a bis 6 dargestellten Trägerstrukturen mit Funktionselementen aufweisen, wobei jeweils in etwa gleichartig geformte Trägerstrukturen und Funktionselemente oder unterschiedlich geformte Trägerstrukturen und Funktionselemente verwendet werden können. Diese Variante ist besonders bevorzugt, wenn die Funktionselemente pharmazeutisch aktive Substanzen beinhalten. Ein derartiges Ausführungsbeispiel ist in Fig. 8 dargestellt. Die in Fig. 3 dargestellten Funktionselemente 28 mit den Trägerstrukturen 26 sind gegenüber liegend an den mäandernden Stegen 5 über die gesamte Wand des Stents verteilt angeordnet.

Alternativ sind derartige Trägerstrukturen an dem distalen und/oder proximalen Ende der Endoprothese und besonders bevorzugt auf einer Umfangslinie angeordnet.

### Bezugszeichenliste:

- 3: Grundgitter
- 4: Steg in Längsrichtung
- 5: zick-zack- oder mäanderförmiger Steg
- 6, 6': fingerförmige Trägerstruktur
- 8: Funktionselement
- 16, 16': Trägerstruktur
- 17, 17': Finger
- 18, 18': Funktionselement
- 26, 26': Trägerstruktur
- 28, 28': Funktionselement
- 36: Trägerstruktur
- 37: Finger
- 38: Funktionselement
- 46: Trägerstruktur
- 47: fraktale Verästelung
- 48: Funktionselement
- 56: Trägerstruktur
- 57: Finger
- 58: Funktionselement
- 59: Bereich
- 66: Trägerstruktur
- 68: Funktionselement
- L: Längsrichtung des Stents
- k: Längsrichtung der Trägerstruktur

## Patentansprüche

1. Endoprothese, insbesondere intraluminale Endoprothese, z.B. ein Stent, mit einem Grundgitter (2) und einem an einer Trägerstruktur (6, 6', 6", 16, 16', 26, 26', 36, 46, 56, 66) befestigten Funktionselement (8, 8', 18, 18', 28, 28', 38, 48, 58, 68), das zumindest in einem Teil seines Volumens eine verglichen mit dem Material des Grundgitters (2) verschiedene Materialzusammensetzung aufweist, **dadurch gekennzeichnet, dass** die Trägerstruktur (6, 6', 6", 16, 16', 26, 26', 36, 46, 56, 66) an ihrem ersten, im Wesentlichen fingerförmigen Ende an dem Grundgitter (2) angeordnet ist und von diesem im Wesentlichen fortsatzartig wegragt, wobei das Funktionselement (8, 8', 18, 18', 28, 28', 38, 48, 58, 68) an einem von dem Grundköper wegragenden Bereich der Trägerstruktur (6, 6', 6", 16, 16', 26, 26', 36, 46, 56, 66) angeordnet ist und diesen Bereich mindestens teilweise umgibt.

2. Endoprothese nach Anspruch 1, **dadurch gekennzeichnet, dass** das Funktionselement (8, 8', 18, 18', 28, 28', 38, 48, 58, 68) das vom Grundgitter (2) wegragende, zweite Ende der Trägerstruktur (6, 6', 6", 16, 16', 26, 26', 36, 46, 56, 66) vollständig umgibt.

3. Endoprothese nach einem der vorhergehenden Ansprüche, **dadurch gekennzeichnet, dass** das Funktionselement (8, 8', 18, 18', 28, 28', 38, 48, 58, 68) im Wesentlichen eine Tropfen-, eine Scheiben- oder eine Kugelform aufweist.

4. Endoprothese nach einem der vorhergehenden Ansprüche, **dadurch gekennzeichnet, dass** das Funktionselement (8, 8', 18, 18', 28, 28', 38, 48, 58, 68) mindestens teilweise aus einem röntgenopaken Material besteht.

5. Endoprothese nach Anspruch 4, **dadurch gekennzeichnet, dass** das röntgenopake Material eines oder mehrere der Elemente aus der Gruppe Gold, Platin, Silber, Wolfram, Iod, Tantal, Yttrium, Niob, Molybdän, Ruthenium, Rhodium, Barium, Lanthan, Cer, Praseodym, Neodym, Samarium, Europium, Gadolinium, Terbium, Dysprosium, Holmium, Erbium, Thulium, Ytterbium, Lutetium, Hafnium, Rhenium, Osmium und Bismut und/oder eine oder mehrere der röntgenopaken Verbindungen aus diesen Elementen und/oder Bariumsulfat, Bismuttrioxid, Bromin, Iodin, Iodid, Titanoxid und Zirkonoxid aufweist.

6. Endoprothese nach einem der vorhergehenden Ansprüche, **dadurch gekennzeichnet, dass** das Funktionselement (8, 8', 18, 18', 28, 28', 38, 48, 58, 68) mindestens eine pharmazeutisch aktive Substanz, vorzugsweise eine Substanz mit antiinflammatorischer, spasmolytischer und/oder antiproliferativer Wirkung enthält.

7. Endoprothese nach Anspruch 6, **dadurch gekennzeichnet, dass** die mindestens eine pharmazeutisch aktive Substanz eine oder mehrere Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Peptide und der Vasodilatatoren (wie beispielsweise Sartane) aufweist.

8. Endoprothese nach einem der vorhergehenden Ansprüche, **dadurch gekennzeichnet, dass** die Trägerstruktur (6, 6', 6", 16, 16', 26, 26', 36, 46, 56, 66) im Wesentlichen fingerförmig ausgebildet ist.

9. Endoprothese nach einem der Ansprüche 1 bis 7, **dadurch gekennzeichnet, dass** die Trägerstruktur (6, 6', 6", 16, 16', 26, 26', 36, 46, 56, 66) einen ersten Finger und an ihrem zweiten Ende mindestens einen zweiten, von dem ersten Finger abzweigenden Finger (17, 17', 37, 57) aufweist.

10. Endoprothese nach einem der vorhergehenden Ansprüche, **dadurch gekennzeichnet, dass** die Trägerstruktur (6, 6', 6", 16, 16', 26, 26', 36, 46, 56, 66) im Bereich ihres zweiten Endes eine Vielzahl fraktaler Verästelungen (47) aufweist.

11. Endoprothese nach einem der Ansprüche 1 bis 9, **dadurch gekennzeichnet, dass** die Trägerstruktur (6, 6', 6", 16, 16', 26, 26', 36, 46, 56, 66) im Bereich ihres zweiten Endes baumförmige Verästelungen (57, 59) ausbildet.

12. Endoprothese nach einem der vorhergehenden Ansprüche, **dadurch gekennzeichnet, dass** die Trägerstruktur (6, 6', 6", 16, 16', 26, 26', 36, 46, 56, 66) zumindest im Bereich ihres zweiten Endes in der radialen Richtung des im Wesentlichen hohlzylinderförmigen Grundgitters (2) eine kleinere Ausdehnung als das Grundgitter (2) aufweist.

13. Endoprothese nach einem der vorhergehenden Ansprüche, **dadurch gekennzeichnet, dass** die Trägerstruktur (6, 6', 6", 16, 16', 26, 26', 36, 46, 56, 66) aus dem Material des Grundgitters (2) besteht.

14. Endoprothese nach einem der Ansprüche 1 bis 12, **dadurch gekennzeichnet, dass** die Trägerstruktur (6, 6', 6", 16, 16', 26, 26', 36, 46, 56, 66) mindestens teilweise aus einem elektrisch isolierenden Material, insbesondere Kunststoff und/oder Keramik besteht.

15. Endoprothese nach einem der vorhergehenden Ansprüche, **dadurch gekennzeichnet, dass** die Trägerstruktur (6, 6', 6", 16, 16', 26, 26', 36, 46, 56, 66) zumindest in dem Bereich, in dem das Funktionselement (8, 8', 18, 18', 28, 28', 38, 48, 58, 68) an der Trägerstruktur (6, 6', 6", 16, 16', 26, 26', 36, 46, 56, 66) angeordnet ist, mit einer elektrisch isolierenden Beschichtung versehen ist.

16. Endoprothese nach einem der vorhergehenden Ansprüche, **dadurch gekennzeichnet, dass** eine Vielzahl von Trägerstrukturen (6, 6', 6", 16, 16', 26, 26', 36, 46, 56, 66) mit Funktionselementen (8, 8', 18, 18', 28, 28', 38, 48, 58, 68) aufweisend eine pharmazeutisch aktive Substanz vorgesehen sind, die gleichmäßig über die gesamte Wand der Gitterstruktur verteilt angeordnet sind.

17. Endoprothese nach einem der vorhergehenden Ansprüche, **dadurch gekennzeichnet, dass** diese eine Vielzahl von Trägerstrukturen (6, 6', 6", 16, 16', 26, 26', 36, 46, 56, 66) mit Funktionselementen (8, 8', 18, 18', 28, 28', 38, 48, 58, 68) mit röntgenopakem Material aufweist, die an dem distalen und/oder an dem proximalen Ende der Endoprothese, vorzugsweise jeweils auf einer Umfangslinie, angeordnet sind.

18. Verfahren zur Herstellung einer Endoprothese nach einem der vorhergehenden Ansprüche, **dadurch gekennzeichnet, dass** das Grundgitter (2) zusammen mit der mindestens einen Trägerstruktur (6, 6', 6", 16, 16', 26, 26', 36, 46, 56, 66) aus einem Hohlzylinder hergestellt wird.

19. Verfahren nach Anspruch 18, **dadurch gekennzeichnet, dass** die zwei- oder dreidimensionalen Grundgitter- und Trägerstrukturen (2, 16, 16', 26, 26', 36, 46, 56, 66) mittels Laserstrahl- oder Wasserstrahlschneiden oder mittels chemischer oder elektrochemischer Ätzverfahren mit und ohne Anwendung lithographischer Techniken hergestellt werden.

20. Verfahren zur Herstellung einer Endoprothese nach einem der Ansprüche 1 bis 17, **dadurch gekennzeichnet, dass** die mindestens eine Trägerstruktur (6, 6', 6", 16, 16', 26, 26', 36, 46, 56, 66) separat vom Grundgitter (2) hergestellt wird und anschließend an dem Grundgitter (2) befestigt wird.

21. Verfahren nach Anspruch 20, **dadurch gekennzeichnet, dass** die separat gefertigte Trägerstruktur (6, 6', 6", 16, 16', 26, 26', 36, 46, 56, 66) mittels Schweißen, Löten, Kleben, mittels einer Presspassung oder einer Klippsverbindung an dem Grundgitter (2) befestigt wird.

22. Verfahren nach Anspruch 20 oder 21, **dadurch gekennzeichnet, dass** die Herstellung der zwei- oder dreidimensionalen Trägerstruktur (6, 6', 6", 16, 16', 26, 26', 36, 46, 56, 66) mittels Laserstrahl- oder Wasserstrahlschneiden oder mittels chemischer oder elektrochemischer Ätzverfahren mit oder ohne Einsatz lithographischer Techniken oder mittels Stanzen, vorzugsweise aus einem plattenförmigen Ausgangskörper erfolgt.

23. Verfahren nach einem der Ansprüche 18 bis 22, **dadurch gekennzeichnet, dass** anschließend an den Herstellungsschritt und ggf. vor dem Anbringen der Trägerstruktur (6, 6', 6", 16, 16', 26, 26', 36, 46, 56, 66) an das Grundgitter (2) das Grundgitter (2) und/oder die Trägerstruktur (6, 6', 6", 16, 16', 26, 26', 36, 46, 56, 66) mittels mechanischer Umformverfahren, beispielsweise Pressen und/oder Biegen, bearbeitet werden.

24. Verfahren nach einem der Ansprüche 18 bis 23, **dadurch gekennzeichnet, dass** das jeweilige Funktionselement (8, 8', 18, 18', 28, 28', 38, 48, 58, 68) mittels Schweißen, Löten, Kleben, Sprühen, Tauchen oder Tunken an der jeweiligen Trägerstruktur (6, 6', 6", 16, 16', 26, 26', 36, 46, 56, 66) befestigt wird.

25. Verfahren nach einem der Ansprüche 18 bis 23, **dadurch gekennzeichnet, dass** das Funktionselements (8, 8', 18, 18', 28, 28', 38, 48, 58, 68) mittels Einbetten des röntgenopaken Materials und/oder der pharmazeutisch aktiven Substanz in eine Matrix aus einem Kohlenstoffpolymer und/oder einem anderen Kunststoff und/oder einer Keramik hergestellt wird, wobei ein Tropfen hiervon an die jeweilige Trägerstruktur (6, 6', 6", 16, 16', 26, 26', 36, 46, 56, 66) angebracht wird und das Polymer des Funktionselements (8, 8', 18, 18', 28, 28', 38, 48, 58, 68) anschließend beispielsweise durch Polymerisation und/oder durch Härten und/oder durch Trocknen und/oder mittels eines Pyrolyseprozesses verfestigt wird.
